(19) Europäisches Patentamt
European Patent Office
Office européen des brevets

(11) Veröffentlichungsnummer: **0 276 432**
**A2**

(12) # EUROPÄISCHE PATENTANMELDUNG

(21) Anmeldenummer: **87118250.7**

(51) Int. Cl.⁴: **C07D 271/06**, A01N 43/82

(22) Anmeldetag: **09.12.87**

(30) Priorität: **12.12.86 CH 4948/86**
**12.12.86 CH 4949/86**
**22.10.87 CH 4134/87**

(43) Veröffentlichungstag der Anmeldung:
**03.08.88 Patentblatt 88/31**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel(CH)**

(72) Erfinder: **Rohr, Otto, Dr.**
**Kilbertweg 19**
**CH-4106 Therwil(CH)**

(74) Vertreter: **Zumstein, Fritz, Dr. et al**
**Bräuhausstrasse 4**
**D-8000 München 2(DE)**

(54) **Schädlingsbekämpfungsmittel.**

(57) Mittel enthaltend als aktive Komponente eine Verbindung der Formel

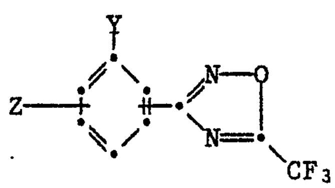

(I),

worin Y für OR' oder HNR" steht, Z Wasserstoff oder $(R_4)_n$ bedeutet, und unter welche als Untergruppen die Verbindungen der Formeln I' und I"

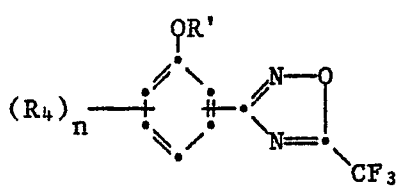

(I')

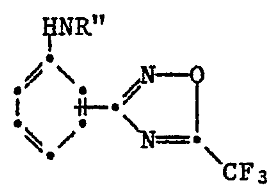

(I")

fallen, in welchen bedeuten:
R' Wasserstoff, $C_1$-$C_4$-Alkyl, Aryl, substituiertes Aryl, $C(X)NR_1'(R_2')$, $C(O)XR_3'$ oder $COR_3'$;
$R_1'$ $C_1$-$C_9$-Alkyl, $C_1$-$C_4$-Alkoxy, $C_2$-$C_4$-Alkenyl, Aryl oder substituiertes Aryl;
$R_2'$ Wasserstoff oder $C_1$-$C_4$-Alkyl;
$R_3'$ $C_1$-$C_{15}$-Alkyl, $C_2$-$C_4$-Alkoxy oder Aryl;
$R_4$ Halogen, $C_1$-$C_4$-Alkoxy oder Nitro; und
n 0, 1 oder 2;
X Sauerstoff oder Schwefel;
R" $C(O)NR''(R_2'')$, $C(S)NR_1''(R_2'')$, $COOR_3''$, $COSR_3''$ oder $COR_3''$;

EP 0 276 432 A2

$R_1$'' $C_1$-$C_4$-Alkyl, $C_1$-$C_4$-Alkoxy oder Aryl;

$R_2$'' Wasserstoff oder $C_1$-$C_4$-Alkyl und

$R_3$'' $C_1$-$C_9$-Alkyl, $C_1$-$C_4$-Haloalkyl, $C_3$-$C_4$-Alkenyl oder Aryl; einschliesslich ihrer Säureadditionssalze

Die Wirkstoffe besitzen mikrobizide Eigenschaften und eignen sich insbesondere zur Bekämpfung phytopathogener Mikroorganismen.

## Schädlingsbekämpfungsmittel

Die vorliegende Erfindung betrifft neue 3-Phenyl-5-trifluormethyl-1,2,4-oxadiazol-Derivate und Schädlingsbekämpfungsmittel, die als Wirkstoff mindestens eine Verbindung der nachstehenden Formel I oder eine Säureadditionsverbindung davon enthalten, die Herstellung dieser Verbindungen oder der sie enthaltenden Mittel sowie Verfahren zur Bekämpfung von Schädlingen, insbesondere die Bekämpfung oder Verhütung des Befalls von Pflazne durch phytopathogene Mikroorganismen.

Die Wirkstoffe entsprechen der Formel I

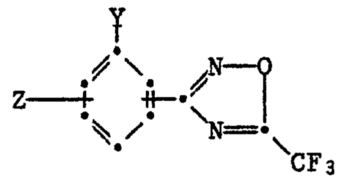

(I),

worin Y für OR' oder HNR" steht, Z Wasserstoff oder $(R_4)_n$bedeutet, und unter welche als Untergruppen die Verbindungen der Formeln I' und I"

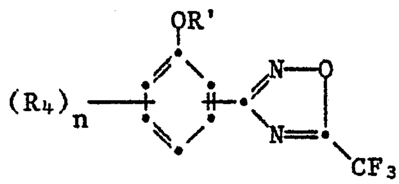

(I')

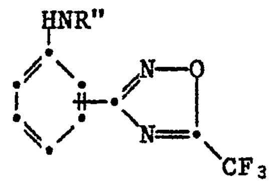

(I")

fallen, in welchen bedeuten:
R' Wasserstoff, $C_1$-$C_4$-Alkyl, Aryl, substituiertes Aryl, C(X)$NR_1$'($R_2$'), C(O)X$R_3$' oder $COR_3$';
$R_1$' $C_1$-$C_9$-Alkyl, $C_1$-$C_4$-Alkoxy, $C_2$-$C_4$-Alkenyl, Aryl oder substituiertes Aryl;
$R_2$' Wasserstoff oder $C_1$-$C_4$-Alkyl;
$R_3$' $C_1$-$C_{15}$-Alkyl, $C_2$-$C_4$-Alkoxy oder Aryl;
$R_4$ Halogen, $C_1$-$C_4$-Alkoxy oder Nitro; und
n 0, 1 oder 2;
X Sauerstoff oder Schwefel;
R" C(O)$NR_1$"($R_2$"), C(S)$NR_1$"($R_2$"), $COOR_3$", $COSR_3$" oder $COR_3$";
$R_1$" $C_1$-$C_4$-Alkyl, $C_1$-$C_4$-Alkoxy oder Aryl;
$R_2$" Wasserstoff oder $C_1$-$C_4$-Alkyl und
$R_3$" $C_1$-$C_9$-Alkyl, $C_1$-$C_4$-Haloalkyl, $C_3$-$C_4$-Alkenyl oder Aryl;
einschliesslich ihrer Säureadditionssalze.

Verbindungen der Formel I' sind neu und Verbingungen der Formel I" sind teilweise neu.

Verbindungen der Formel Ia' stellen eine bevorzugte Gruppe dar:

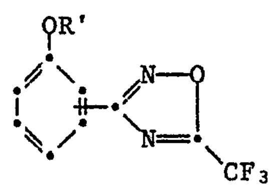

(Ia'),

in welcher
R' für Wasserstoff, $C_1$-$C_4$-Alkyl, Aryl, substituiertes Aryl, C(X)$NR_1$'($R_2$'), C(O)X$R_3$' oder $COR_3$' steht;
$R_1$' $C_1$-$C_9$-Alkyl, $C_1$-$C_4$-Alkoxy, $C_2$-$C_4$-Alkenyl, Aryl oder substituiertes Aryl;
$R_2$' Wasserstoff oder $C_1$-$C_4$-Alkyl;
$R_3$' $C_1$-$C_{15}$-Alkyl, $C_2$-$C_4$-Alkenyl oder Aryl
und

X Sauerstoff oder Schwefel darstellt, einschliesslich ihrer Säureadditionsalze.

Eine weitere Gruppe stellen Verbindungen der Formel I' dar, in welcher n 1 oder 2 bedeutet.

Verbindungen der Formel I", bei denen der Rest

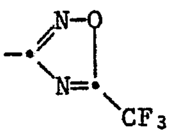

in 4-Stellung des Phenylrings steht und R" die unter Formel I" angegebenen Bedeutungen besitzt, sind neu. Diese neuen Verbindungen werden als Teil des Verbindungsumfangs der Formel I" unter der Formel Ia"

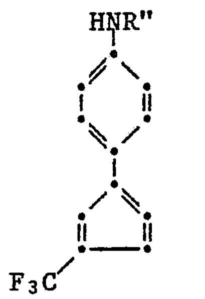

(Ia"),

in welcher R" die unter Formel I angegebenen Bedeutungen besitzt, zusammengefasst. Die neuen Verbindungen der Formel Ia" einschliesslich ihrer Säureadditionssalze stellen einen Teil der Erfindung dar.

Unter Alkyl selbst oder als Bestandteil eines anderen Substituenten sind je nach Zahl der angegebenen Kohlenstoffatome gerad-oder verzweigtkettige Gruppen, beispielsweise folgende, zu verstehen: Methyl, Ethyl, Propyl, Butyl, Pentyl, Hexyl, Heptyl, Octyl oder Nonyl bis Pentadecyl sowie ihre Isomeren wie z.B. Isopropyl, Isobutyl, tert.-Butyl, sek.-Butyl oder Isopentyl.

Alkenyl steht z.B. für Propenyl-(1), Allyl, Butenyl-(1), Butenyl-(2) oder Butenyl-(3).

Halogen selbst oder als Bestandteil eines anderen Substituenten bedeutet Fluor, Chlor, Brom oder Jod, vorzugsweise Fluor, Chlor oder Brom.

Unter Aryl sind Phenyl oder Naphthyl, vorzugsweise Phenyl, zu verstehen. Als Substituenten des Aryl kommen in Frage Halogen, $C_1$-$C_4$-Alkyl, insbesondere Methyl, oder $C_1$-$C_2$-Haloalkyl mit 1 bis 3 Halogen, insbesondere Trifluormethyl.

Als Salzbildner mit Verbindungen der Formel I kommen alle organischen und anorganischen Säuren in Frage soweit sie pflanzenphysiologisch verträgliche Salze bilden.

Beispiele salzbildender Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, Phosphorige Säure, Salpetersäure und organische Säuren wie Essigsäure, Trifluoressigsäure, Trichloressigsäure, Propionsäure, Weinsäure, Glykolsäure, Thiocyansäure, Milchsäure, Bernsteinsäure, Zitronesäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure. Diese Säuren werden nach an sich bekannten Methoden an die betreffenden freien Verbindungen der Formel I addiert.

Die Verbindungen der Formel I sind bei Raumtemperatur stabil. Sie lassen sich auf dem Agrarsektor oder verwandten Gebieten insbesondere präventiv und kurativ zur Bekämpfung von pflanzenschädigenden Mikroorganismen einsetzen. Die Wirkstoffe der Formel I zeichnen sich in weiten Anwendungskonzentrationen durch eine sehr gute fungizide Wirkung und problemlose Anwendung aus. Darüber hinaus besitzen die Verbindungen weitere pestizide Eigenschaften. So können sie auch zur Bekämpfung von Nematoden, insbesondere von pflanzenschädigenden Nematoden, und fressenden und saugenden pflanzenschädigenden Insekten eingesetzt werden.

Aufgrund ihrer ausgeprägten pestiziden, insbesondere mikrobiziden Wirksamkeit sind der Formel Ia' insbesondere diejenigen Verbindungen bevorzugt, in welchen

a) R' für Wasserstoff, Phenyl, substituiertes Phenyl, $C(X)NR_1'(R_2')$, $C(O)XR_3'$ oder $COR_3'$ steht;
$R_1'$ $C_1$-$C_9$-Alkyl, $C_1$-$C_4$-Alkoxy, $C_2$-$C_4$-Alkenyl oder Phenyl;
$R_2'$ Wasserstoff oder C-$C_4$-Alkyl;
$R_3'$ $C_1$-$C_{15}$-Alkyl, $C_2$-$C_4$-Alkenyl oder Phenyl bedeuten; und
X Sauerstoff oder Schwefel darstellt, oder
b) der Rest

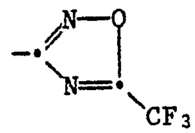

in 4-Stellung des Phenylrings steht und welche gleichzeitig folgende Substituenten oder Kombinationen dieser Substituenten untereinander aufweisen:
R' steht für Wasserstoff, Phenyl, durch Halogen oder Methyl substituiertes Phenyl, $C(X)NR_1'(R_2')$, $C(O)XR_3'$ oder $COR_3'$, wobei $R_1'$ $C_1$-$C_4$-Alkyl, $C_1$-$C_2$-Alkoxy, Allyl oder Phenyl; $R_2'$ Wasserstoff oder $C_1$-$C_2$-Alkyl; $R_3'$ $C_1$-$C_9$-Alkyl, $C_2$-$C_4$-Alkenyl oder Phenyl bedeuten; und
X Sauerstoff oder Schwefel darstellt; oder
R' für Wasserstoff, $C_1$-$C_4$-Alkyl, Phenyl substituiertes Phenyl, $C(X)NR_1'(R_2')$, $C(O)XR_3'$ oder $COR_3'$ steht, wobei $R_1'$ $C_1$-$C_4$-Alkyl, $C_1$-$C_4$-Alkoxy, Allyl oder Phenyl; $R_2'$ Wasserstoff oder $C_1$-$C_4$-Alkyl; $R_3'$ $C_1$-$C_9$-Alkyl, $C_2$-$C_4$-Alkenyl oder Phenyl bedeuten; und X Sauerstoff oder Schwefel darstellt.

Folgende erfindungsgemässe Einzelverbindungen der Formel Ia' sind wegen ihrer biologischen Aktivität bevorzugt:
3-(4-Methylaminocarbonyloxyphenyl)-5-trifluormethyl-1,2,4-oxadiazol;
3-(4-Isopropylaminocarbonyloxyphenyl)-5-trifluormethyl-1,2,4-oxadiazol;
3-(4-Ethylaminocarbonyloxyphenyl)-5-trifluormethyl-1,2,4-oxadiazol;
3-(4-Allylaminocarbonyloxyphenyl)-5-trifluormethyl-1,2,4-oxadiazol;
3-[4-(2-Chlor-4-trifluormethylphenoxy)-phenyl]-5-trifluormethyl-1,2,4-oxadiazol;
3-[Methoxy-(methyl)-aminocarbonyloxyphenyl]-5-trifluormethyl-1,2,4-oxadiazol;
3-(4-Hexanoyloxyphenyl)-5-trifluormethyl-1,2,4-oxadiazol;
3-[4-(phenylaminocarboxy)-phenyl]-5-trifluormethyl-1,2,4-oxadiazol;
3-[4-(2-Methyl-3-chlorphenylaminocarboxy)-phenyl]-5-trifluormethyl-1,2,4-oxadiazol;
3-[4-(3-Ethylphenylaminocarboxyphenyl)]-5-trifluormethyl-1,2,4-oxadiazol.

Von den Verbindungen der Formel I" sind diejenigen bevorzugt, die unter die Formel I"

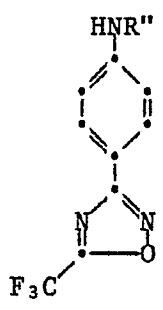

(Ia"),

fallen, in welcher
R" für die Gruppen $C(O)NR_1"(R_2")$, $C(S)NR_1")$, $COOR_3"$, $COSR_3"$ oder $COR_3"$ steht, worin
$R_1"$ $C_1$-$C_4$-Alkyl, $C_1$-$C_4$-Alkoxy oder Aryl;
$R_2"$ Wasserstoff oder $C_1$-$C_4$-Alkyl und
$R_3"$ $C_1$-$C_9$-Alkyl, $C_1$-$C_4$-Haloalkyl, $C_3$-$C_4$-Alkenyl oder Aryl bedeuten.

Darüber hinaus sind solche Verbindungen der Formel Ia" bevorzugt, die folgende Substituenten oder Kombinationen dieser Substituenten untereinander aufweisen:
R" steht für $C(O)NR_1"(R_2")$, $C(S)R_1"(R_2")$, $COOR_3"$, $COSR_3"$ oder $COR_3"$, wobei $R_1"$ $C_1$-$C_2$-Alkyl, $C_1$-$C_2$-Alkoxy oder Phenyl, $R_2"$ Wasserstoff oder $C_1$-$C_2$-Alkyl und $R_3"$ $C_1$-$C_3$-Alkyl, $C_1$-$C_2$-Haloalkyl, $C_3$-$C_4$-Alkenyl oder Phenyl bedeuten.

Ferner sind solche Mittel bevorzugt, deren Wirkstoffe Verbindungen der Formel I" sind, die folgende Substituenten oder Kombinationen dieser Substituenten aufweisen:

R″ steht für $C(O)NR_1''(R_2'')$, $C(S)NR_1''(R_2'')$, $COR_3''$, $COOR_3''$, oder $COSR_3''$, wobei $R_1''$ $C_1$-$C_2$-Alkyl, $C_1$-$C_2$-Alkoxy oder Phenyl, $R_2''$ Wasserstoff oder $C_1$-$C_2$-Alkyl und $R_3''$ $C_1$-$C_3$-Alkyl, $C_1$-$C_2$-Haloalkyl, $C_3$-$C_4$-Alkenyl oder Phenyl bedeuten.

Folgende erfindungsgemässe Einzelverbindungen sind wegen ihrer biologischen Aktivität bevorzugt:
3-[4-(3-Methyl-3-methoxyureido)-phenyl]-5-trifluormethyl-1,2,4-oxodiazol;
3-[4-(3,3-Dimethylureido)-phenyl]-5-trifluormethyl-1,2,4-oxadiazol;
3-(4-Methyoxycarbonylaminophenyl)-5-trifluormethyl-1,2,4-oxadiazol;
3-(4-Ethoxycarbonylaminophenyl)-5-trifluormethyl-1,2,4-oxadiazol;
3-(4-Isopropoxycarbonylaminophenyl)-5-trifluormethyl-1,2,4-oxadiazol;
3-(4-Phenyloxycarbonylaminophenyl)-5-trifluormethyl-1,2,4-oxadiazol;
3-(4-Acetylaminophenyl)-5-trifluormethyl-1,2,4-oxadiazol;
3-(4-Allyloxycarbonylaminophenyl)-5-trifluormethyl-1,2,4-oxadiazol;
3-[4-(2-Chlorethyloxycarbonylamino)-phenyl]-5-trifluormethyl-1,2,4-oxadiazol;
3-[4-(3-Methylthioureido)-phenyl]-5-trifluormethyl-1,2,4-oxadiazol;
3-[4-(3,3-Dimethylthioureido)-phenyl]-5-trifluormethyl-1,2,4-oxadiazol;
3-[4-(3-Methyl-3-methoxythioureido)-phenyl]-5-trifluormethyl-1,2,4-oxadiazol;
3-(4-Ethylthiocarbonylaminophenyl)-5-trifluormethyl-1,2,4-oxadiazol;
3-(4-Ethylcarbonylaminophenyl)-5-trifluormethyl-1,2,4-oxadiazol;
3-(4-Chlormethylcarbonylaminophenyl)-5-trifluormethyl-1,2,4-oxadiazol.

Folgende Verbindungen sind als Wirkstoffe von erfindungsgemässen Mitteln wegen ihrer biologischen Aktivität bevorzugt:
3-[3-(3,3-Dimethylureido)-phenyl]-5-trifluormethyl-1,2,4-oxadiazol;
3-[3-(3-Methyl-3-methoxyureido)-phenyl]-5-trifluormethyl-1,2,4-oxadiazol;
3-[3-(3-Methylureido)-phenyl]-5-trifluormethyl-1,2,4-oxadiazol;
3-(3-Ethoxycarbonylaminophenyl)-5-trifluormethyl-1,2,4-oxadiazol;
3-(3-Chlormethoxycarbonylaminophenyl)-5-trifluormethyl-1,2,4-oxadiazol;
3-(3-Methoxycarbonylaminophenyl)-5-trifluormethyl-1,2,4-oxadiazol;
3-(3-Isopropoxycarbonylaminophenyl)-5-trifluormethyl-1,2,4-oxadiazol;
3-(3-Phenoxycarbonylaminophenyl)-5-trifluormethyl-1,2,4-oxadiazol;
3-(3-Ally(oxycarbonylaminophenyl)-5-trifluormethyl-1,2,4-oxadiazol;
3-[3-(2-Chlorethoxycarbonylamino)-phenyl]-5-trifluormethyl-1,2,4-oxadiazol.

Die Verbindungen der Formel I werden wie folgt hergestellt:

1. Verbindungen der Formel I′

Ein Oximderivat der Formel II′

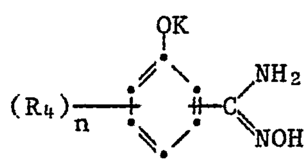

(II')

wird mit Trifluoressigsäureanhydrid zu einem Phenolderivat der Formel Ib′

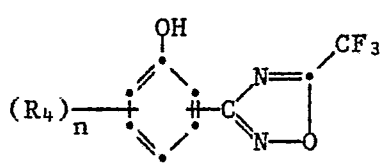

(Ib')

umgesetzt, welches dann zur weiteren Substitution

1.1 mit einem Carbamoylchlorid der Formel III′

$R_1'(R_2')N$-CXCl (III′)

oder

1.2 mit einem Chlorameisensäureester der Formel IIIa′

$R_3'X\overset{O}{\overset{\|}{C}}$ -Cl (IIIa′)

oder

1.3 mit einem Carbonsäurechlorid der Formel IIIb′

$R_3'-\overset{O}{\overset{\|}{C}}$ -Cl (IIIb′)

oder

1.4 mit Dialkylsulfat oder Alkyhalogenid, vorzugsweise Alkylbromid oder Alkyljodid, mit 1 bis 4 Kohlenstoffatomen in den Alkylgruppen in Gegenwart einer Base als Säureakzeptor zur Reaktion gebracht wird, wobei X Sauerstoff oder Schwefel bedeutet und $R_1'$, $R_2'$, $R_3'$, $R_4$ und n die unter Formel I angegebenen Bedeutungen besitzen,

oder

1.5 ein Oximderivat der Formel IIa′

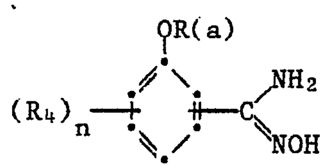

(IIa′)

wird mit Trifluoressigsäureanhydrid zu einem Phenolderivat der Formel Ic′

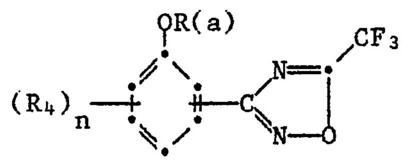

(Ic′) ,

worin R(a) Aryl oder substituiertes Aryl darstellt und $R_4$ und n die unter Formel I angegebenen Bedeutungen besitzen, umgesetzt.

2. Verbindungen der Formel I″

Ein Anilinderivat der Formel II″

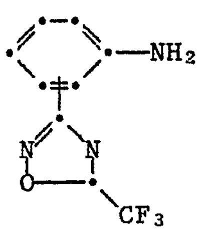

(II″)

wird

2.1 mit einem Carbamoylchlorid der Formel III″

$R_1''(R_2'')N$-CXCl (III″)

oder

2.2 mit einem Chlorameisensäureester der Formel IIIa″

$R_3''X\overset{O}{\overset{\|}{C}}$ -Cl (IIIa″)

oder

2.3 mit einem Carbonsäurechlorid der Formel IIIb″

$R_3''-\overset{O}{\overset{\|}{C}}$ -Cl (IIIb″)

in einem inerten Lösungsmittel in Gegenwart einer Base als Säureakzeptor

oder

2.4 mit einem Isocyanatderivat der Formel IV

$R_1''-N=C=X$ (IV)

in einem inerten Lösungsmittel umgesetzt, wobei X Sauerstoff oder Schwefel bedeutet und $R_1''$, $R_2''$ und $R_3''$ die unter Formel I'' angegebenen Bedeutungen besitzen.

In den Verfahrensvarianten (1.1) bis (1.4) und (2.1) bis (2.3) können als Basen alle geeigneten Säurebindemittel eingesetzt werden, wie Amine, insbesondere tertiäre Amine, sowie Alkali-und Erdalkaliverbindungen. Beispielhaft seien dafür erwähnt die Hydroxide, Oxide und Karbonate des Lithiums, Natriums, Kaliums, Magnesiums, Calciums und Bariums, ferner weitere basische Verbindungen wie Trimethylamin, Triethylamin, Tripropylamin, Tributylamin, Tribenzylamin, Tricyclohexylamin, Triamylamin, Trihexylamin, N,N-Dimethylanilin, N,N-Dimethyltoluidin, N,N-Dimethyl-p-amino-pyridin, N-Methylpyrrolidin, N-Methyl-piperidin, N-Methylimidazol, N-Methyl-pyrrol, N-Methylmorpholin, N-Methylhexamethylenimin, Pyridin, Chinolin, alpha-Picolin, β-Picolin, Isochinolin, Pyrimidin, Acridin, N,N,N',N'-Tetra-methylethylendiamin, N,N,N',N'-Tetraethylethylendiamin, Chinoxalin, N-Propyldiisopropylamin, N,N-Dimethylcyclohexylamin, 2,6-Lutidin, 2,4-Lutidin, Triethylendiamin.

In den Verfahrensvarianten (1.1) bis (1.5) und (2.1) bis (2.4) werden in Anspassung an die jeweiligen Reaktionsbedingungen inerte Lösungs-und Verdünnungsmittel verwendet. Als Beispiele sind zu nennen:

Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Chlornaphthalin, Dichlornaphthalin, Tetrachlorkohlenstoff, Trichlorethan, Trichlorethylen, Pentachlorethan, Difluorbenzol, 1,2-Dichlorethan, 1,1-Dichlorethan, 1,2-cis-Dichlorethylen, Chlorbenzol, Fluorbenzol, Brombenzol, Jodbenzol, Dichlorbenzol, Dibrombenzol, Chlortoluol, Trichlorbenzol; Ether, wie Ethylpropylether, Methyl-tert.-butylether, n-Butylethylether, Di-n-butylether, Di-isobutylether, Diisoamylether, Diisopropylether, Anisol, Phenetol, Cyclohexylmethylether, Diethyl ether, Ethylenglykoldimethylether, Tetrahydrofuran, Dioxan, Thioanisol, Dichlordiethylether; Nitrokohlenwasserstoffe wie Nitromethan, Nitroethan, Nitrobenzol, Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, wie Heptan, Pinan, Nonan, Cymol, Benzinfraktionen innerhalb eines Siedepunktintervalles von 70° bis 190°C, Cyclohexan, Methylcyclohexan, Dekalin, Petrolether, Hexan, Ligroin, Trimethylpentan, Octan; Ester wie Ethylacetat, Acetessigester, Isobutylacetat; Amide, z.B. Formamid, Methylformamid, Dimethylformamid; Ketone, wie Aceton, Methylethylketon, gegebenenfalls auch Wasser. Auch Gemische der genannten Lösungs-und Verdünnungsmittel kommen in Betracht.

Die Reaktionstemperaturen betragen in den Verfahrensvarianten (1.1) bis (1.5) 0° bis 180°C, vorzugsweise 40° bis 120°C, und speziell in der Variante (1.4) 0° bis 100°C, vorzugsweise 20°C bis 80°C, und in den Verfahrensvarianten (2.1) bis (2.4) 20° bis 180°C, vorzugsweise 40° bis 120°C.

Ein Teil der unter die Formel I fallenden Wirkstoffe sind aus der deutschen Offenlegungsschrift 28 01 509 bekannt. Sie werden darin als Wirkstoffe von herbiziden Mitteln vorgeschlagen.

Es wurde nun überraschend gefunden, dass die erfindungsgemässen Verbindungen der Formel I und die Mittel, die diese Verbindungen als Aktivstoffe enthalten, ein für praktische Bedürfnisse sehr günstiges Mikrobizid-Spektrum gegen phytopathogene Pilze und Bakterien aufweisen. Sie besitzen sehr vorteilhaft kurative, systemische und insbesondere präventive Eigenschaften und lassen sich zum Schutz von zahlreichen Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe der Formel I sind gegen die den folgenden Klassen angehörenden phytopathogenen Pilze wirksam: Fungi imperfecti (z.B. Botrytis, Helminthosporium, Fusarium Septoria, Cercospora und Alternaria); Basidiomyceten (z.B. die Gattungen Hemileia, Rhizoctonia, Puccinia); ferner wirken sie gegen die Klasse der Ascomyceten (z.B. Venturia, Podosphaera, Erysiphe, Monilinia, Uncinula). Ueberdies wirken die Verbindungen der Formel I systemisch. Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Neben der mikrobiziden Wirksamkeit weisen die Wirkstoffe der Formel I nematizide Eigenschaften auf, was sie insbesondere zur Bekämpfung von Pflanzennematoden geeignet macht. Zu diesem Zweck können die erfindungsgemässen Mittel kurativ, präventiv oder systemisch eingesetzt werden. Dabei entfalten sie eine breit gefächerte Aktivität gegen die verschiedenen Nematodenspecies und werden somit den Erfordernissen der Praxis gerecht.

In den weiter unten angegebenen Aufwandmengen zeichnen sich die erfindungsgemässen Wirkstoffe durch besonders gute Pflanzenverträglichkeit aus, auch jene, die in der DE-OS 28 01 509 vorgeschlagen sind.

Die Erfindung betrifft auch die Verwendung der Verbindungen der Formel I zur Bekämpfung phytopathogener Mikroorganismen, insbesondere pflanzenschädigender Pilze bzw. die präventive Verhütung eines Befalls von Pflanzen.

Darüber hinaus schliesst die vorliegende Erfindung auch die Herstellung agrochemischer Mittel ein, die gekennzeichnet ist durch das innige Vermischen der Aktivsubstanz mit mindestens einem hierin beschriebenen Formulierungshilfsstoff. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung z.B. folgende Pflanzenarten: Getreide: (Weizen, Gerste, Roggen, Hafer, Reis, Sorghum und Verwandte); Rüben: (Zucker-und Futterrüben); Kern-, Stein-und Beerenobst: (Aepfel, Birnen, Pflaumen, Pfirsiche, Mandeln, Kirschen, Erd-, Him-und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Grapefruit, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen-und Naturkautschukgewächse sowie Zierpflanzen (Blumen, Sträucher, Laubbäume und Nadelbäume wie Koniferen). Die Aufzählung stellt keine Limitierung dar.

Wirkstoffe der Formel I werden üblicherweise in Form von Zusammensetzungen verwendet und können gleichzeitig oder nacheinander mit weiteren Wirkstoffen auf die Umgebung der zu behandelnden Pflanze oder die Pflanze selbst gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz, Haft-, Verdickungs-, Binde-oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt z.B. in Form von Granulat (Bodenapplikation). Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angstrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl-oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl-oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden, in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisat zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation-und/oder anionaktive Tenside mit guten Emulgier-, Dispergier-und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali-oder gegebenenfalls substituierten Ammoniumsalze von höheren Fettsäuren ($C_{10}$-$C_{22}$), wie z.B. die Na-oder K-Salze der Oel-oder Stearinsäure, oder von natürlichen Fettsäuregemischen, die z.B. aus Kokosnuss-oder Talgöl gewonnen werden können. Ferner sind auch die Fettsäure-methyllaurinsalze zu erwähnen.

Häufiger werden jedoch sog. synthetische Tenside verwendet, insbesondere Fettalkoholsulfonate, Fettalkoholsulfate, sulfonierte Benzimidazolderivate oder Alkylsulfonate.

Die Fettalkoholsulfonate oder -sulfate liegen in der Regel als Alkali-, Erdalkali-oder gegebenenfalls substituierte Ammoniumsalze vor und weisen einen Alkylrest mit 8 bis 22 C-Atomen auf, wobei Alkyl auch den Alkylteil von Acylresten einschliesst, z.B. Das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und eine Fettsäurerest mit 8-22 C-Atomen. Alkylarylsulfonate sind z.B. die Na-, Ca-oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl-oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartiger agrochemische Mittel sind ein Bestandteil der vorliegenden Erfindung.

Die nachfolgenden Beispiele dienen zur näheren Erläuterung der Erfindung, ohne dieselbe einzuschränken.

1. Herstellungsbeispiele

Beispiel 1.1:

a) Herstellung von

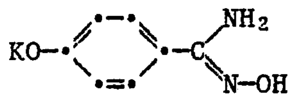

(A)

65,5 g (0,55 Mol) 4-Hydroxibenzonitril und 133,8 g Hydroxylaminhydrochlorid werden in 600 ml 80%igem Ethanol vorgelegt und während 20 Min. portionenweise 296,5 g (2,145 Mol) Kaliumcarbonat zugegeben. Das Reaktionsgemisch wird während 12 Stdn am Rückfluss gerührt, auf Raumtemperatur abgekühlt und abfiltriert. Der Filterkuchen wird mit heissem Ethanol gewaschen und das Filtrat eingedampft. Die hellrote Substanz wird mit Aceton digeriert, abfiltriert und getrocknet. Man erhält 79,6 g (= 76,1 % d. Th.) der Titelverbindung (A) (Smp. 158°-160°C).

b) Herstellung von

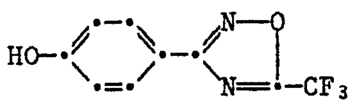

(B)

152,2 g (0,8 Mol) der Verbindung (A) werden in 1,5 Liter Tetrahydrofuran suspendiert und während 1 Stunde 170 ml (~ 1,2 Mol) Trifluoressigsäureanhydrid zugetropft, wobei sich die Suspension unter exothermer Reaktion löst. Durch Kühlen hält man die Innentemperatur bei ca. 40°C. Anschliessend wird bei Rautemperatur während 2 Stdn gerührt und die Reaktionsmischung in Eis/Wasser gegossen. Das ausgeschiedene Oel wird mit Toluol/Essigsäureethylester extrahiert. Der ölige Rückstand kristallisiert beim Stehenlassen aus. Man erhält 102,9 g (47,4 % d. Th.) der Titelverbindung (B) mit einem Smp. von 66°-73°C.

c) Herstellung von

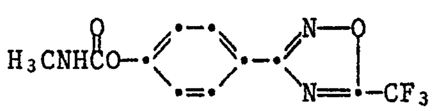

(C)

46 g (0,2 Mol) der Verbindung (B) werden zusammen mit 0,5 g Triäthylendiamin in 300 ml Toluol vorgelegt. Dazu tropft man bei Raumtemperatur 23 ml Methylisocyanat (leicht exotherm) wobei vollständige Lösung eintritt. Nach ca. 15 Min. Rühren beginnt das Reaktionsprodukt auszufallen, wobei die Temperatur bis 30°C ansteigt. Nach Abschluss des Rührvorganges über Nacht werden 170 ml Cyclohexan zugegeben und dann abfiltriert. Man erhält 55,4 g (96,5 % d. Th.) der Titelverbindung mit einem Smp. von 180°-185°C.

Beispiel 1.2: Herstellung von

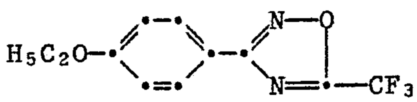

13,8 g (0,06 Mol) 3-(4-Hydroxyphenyl)-5-trifluormethyl-1,2,4-oxadiazol werden in 100 ml 2-Butanon vorgelegt und mit 9,9 g (0,072 Mol) Kaliumcarbonat versetzt. Dazu werden 8,6 ml (0,066 Mol) Diäthylsulfat getropft. Die Suspension wird während 18 Stunden bei 80°C gerührt und nach dem Abkühlen auf Raumtemperatur abfiltriert. Nach Abdestillieren des Lösungsmittels erhält man 13,7 g gelbes Oel, das beim Stehenlassen kristallisiert. Smp. 48-51°C.

Beispiel 1.3:

a) Herstellung von

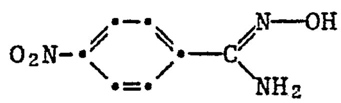

(A)

148,1 g (1 Mol) 4-Nitrobenzonitril werden mit 139 g (2 Mol) Hydroxylaminhydrochlorid in 1300 ml Methanol vorgelegt. Dazu gibt man innerhalb von 20 Minuten portionenweise 80 g (2 Mol) Natriumhydroxid (exotherm). Die gelbe Suspension wird während 3,5 Stunden auf Rückflusstemperatur erwärmt und anschliessend heiss filtriert. Durch Zugabe von Eis/Wasser kristallisiert die Titelverbindung (A) aus.
Aubeute: 162,9 g (= 89,9 % d. Th.) Smp. 168°-170°C.

b) Herstellung von

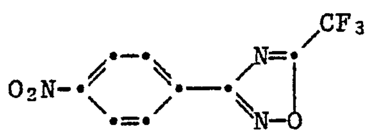

(B)

74,3 g (0,41 Mol) der Verbindung (A) werden in 1400 ml Tetrahydrofuran gelöst und innerhalb 1 Stunde 98,3 g (0,47 Mol) Trifluoressigsäureanhydrid zugetropft. Die Temperatur steigt auf 35°C. Nach 2 Stunden Rühren bei Raumtemperatur wird auf das halbe Volumen eingedampft, mit der doppelten Mengen Wasser verdünnt und mit Toluol/Essigsäureethylester extrahiert. Der Extrakt wird eingedampft und der Rückstand aus Ethanol/Wasser umkristallisiert. Man erhält Kristalle der Titelverbindung (B).
Ausbeute: 72,8 g (= 63,5 % d. Th.) Smp. 62°-63°C.

c) Herstellung von

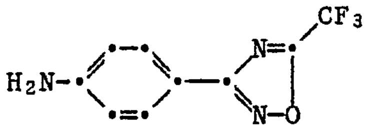

(C)

262.8 g (1,02 Mol) der Verbindung (B) werden in 2200 ml Ethanol bei 35°C gelöst und eine Suspension von 109,2 g Ammoniumchlorid (2 Mol) in 120 ml Wasser zugegeben. Innerhalb von 30 Minuten werden 467,2 g Zinkpulver (7,2 Mol) portionenweise eingestreut und anschliessend das Reaktionsgemisch 10 Stunden bei Rückflusstemperatur und nochmals 10 Stunden bei Raumtemperatur gerührt. Das Zinkpulver wird abfiltriert und das Lösungsmittel abdestilliert. Das erhaltene Rohprodukt wird in Toluol/Essigester 1:2 gelöst, von unlöslichen Nebenprodukten abfiltriert und die Lösung am Rotationsverdampfer eingeengt. Die ausgeschieden Kristalle werden abfiltriert. Man erhält die Titelverbindung (C).
Ausbeute: 142,4 g (= 60,9 % d.Th.) Smp. 53°-58°C.

d) Herstellung von

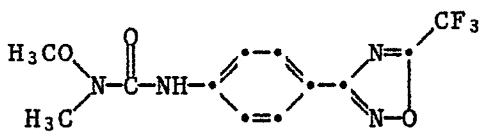

(D)

141,2 g (0,616 Mol) der Verbindung (C) werden bei Raumtemperatur in 480 ml Pyridin gelöst und innerhalb 5 Minuten 135,9 g (1,1 Mol) N-Methyl-N-methoxycarbamoylchlorid zugetropft, wobei die Temperatur auf 53°C ansteigt. Nach 1 Stunde Rühren bei Rautmemperatur wird die Reaktionsmischung in Eis/Wasser gegossen und abfiltriert. Man erhält die Titelverbindung (D).
Ausbeute: 177,4 g (= 91,1 % d.Th.) Smp. 88°-90°C.

Analog dem vorstehend beschriebenen Beispiel lassen sich die nachfolgend aufgeführten Verbindungen herstellen.

Tabelle 1

Verbindungen der Formel

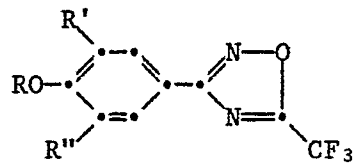

| Verb.Nr. | R | R' | R" | Smp. (°C) |
|---|---|---|---|---|
| 1.1 | $C(O)NHCH_3$ | H | H | 180-185 |
| 1.2 | $C_6H_3Cl(2)CF_3(4)$ | H | H | 59-61 |
| 1.3 | H | I | I | 110-112 |
| 1.4 | H | H | H | 68-72 |
| 1.5 | $CH_3$ | H | H | 41-44 |
| 1.6 | $C(O)NCH_3(CH_3O)$ | H | H | 46-49 |
| 1.7 | $C(O)N(CH_3)_2$ | H | H | 62-66 |
| 1.8 | $C(O)C_5H_{11}$ | H | H | 67-69 |
| 1.9 | $C(O)C_{15}H_{31}$ | H | H | 62-64 |
| 1.10 | $C(O)C_7H_{15}$ | H | H | 42-43 |
| 1.11 | $C(O)C_9H_{19}$ | H | H | 35-37 |
| 1.12 | $C(O)C_6H_{13}$ | H | H | 51-53 |
| 1.13 | $C_2H_5$ | H | H | 48-51 |
| 1.14 | $C(O)NHCH(CH_3)_2$ | H | H | 152-154 |
| 1.15 | $C(O)NHC_2H_5$ | H | H | 149-151 |
| 1.16 | $C(O)NHCH_2-CH=CH_2$ | H | H | 123-125 |
| 1.17 | $C(O)NH-C_6H_5$ | H | H | 133-135 |
| 1.18 | $C(O)NHC_6H_3(CF_3)_2(3,5)$ | H | H | 141-143 |
| 1.19 | $C(O)NHC_6H_3CH_3(2)Cl(3)$ | H | H | 179-181 |
| 1.20 | $C(O)NHC_6H_4C_2H_5(3)$ | H | H | 114-116 |
| 1.21 | H | Cl | Cl | 103-110 |
| 1.22 | $C(O)NHCH_3$ | Cl | Cl | 144-145 |

Tabelle 1 (Fortsetzung)

| Verb.Nr. | R | R' | R" | Smp. (°C) |
|---|---|---|---|---|
| 1.23 | H | Br | Br | 96-99 |
| 1.24 | $C(O)NHCH_3$ | Br | Br | 161-165 |
| 1.25 | $C(O)NHCH_3$ | $OCH_3$ | Br | 164-167 |
| 1.26 | H | $OCH_3$ | Br | 179-182 |
| 1.27 | $C(O)NHCH_3$ | $OCH_3$ | $NO_2$ | 109-112 |
| 1.28 | H | $OCH_3$ | H | 102-103 |
| 1.29 | H | $NO_2$ | $NO_2$ | 94-97 |
| 1.30 | $C(O)NHCH_3$ | $NO_2$ | $NO_2$ | 114-116 |
| 1.31 | H | $OCH_3$ | $NO_2$ | 98-102 |
| 1.32 | $C(O)NHCH_3$ | $OCH_3$ | H | 166-168 |
| 1.33 | $C(O)OCH_3$ | H | H | 65-66 |
| 1.34 | $C(O)OC_2H_5$ | H | H | 48-49 |
| 1.35 | $C(O)OCH(CH_3)_2$ | H | H | 63-64 |
| 1.36 | $C(O)SC_2H_5$ | H | H | |
| 1.37 | $C(S)NHCH_3$ | H | H | |
| 1.38 | $C(S)N(CH_3)_2$ | H | H | |
| 1.39 | $C_6H_4F(4)$ | H | H | |
| 1.40 | $C(O)SC_2H_5$ | H | H | 45-47 |
| 1.41 | $C(S)N(CH_3)_2$ | H | H | 92-96 |
| 1.42 | $C(O)NHC_3H_7$ | H | H | 130-132 |
| 1.43 | $C(O)NHC(CH_3)_3$ | H | H | 90-92 |
| 1.44 | $C(O)NHC_4H_9$ | H | H | 128-131 |

Tabelle 2

Verbindungen der Formel

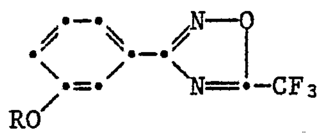

| Verb.Nr. | R | phys. Daten |
|---|---|---|
| 2.1 | H | Smp. 64-66°C |
| 2.2 | $C(O)NHCH_3$ | Smp. 74-77°C |
| 2.3 | $C(O)OCH_3$ | $n_D^{25,5}$ 1,4820 |
| 2.4 | $C(O)OC_2H_5$ | $n_D^{25}$ 1,4793 |
| 2.5 | $C(O)OCH(CH_3)_2$ | $n_D^{25}$ 1,4736 |
| 2.6 | $C(O)SC_2H_5$ | $n_D^{25}$ 1,5170 |
| 2.7 | $C(S)NHCH_3$ | |
| 2.8 | $C(S)N(CH_3)_2$ | Smp. 95-98°C |
| 2.9 | $C_6H_4F(4)$ | Oel |
| 2.10 | $C(O)NHC_3H_7$ | Smp. 83-84°C |
| 2.11 | $C(O)NHCH(CH_3)_2$ | Smp. 103-105°C |
| 2.12 | $C(O)NHC(CH_3)_3$ | Smp. 103-105°C |
| 2.13 | $C(O)NHC_4H_9$ | Smp. 56-58°C |

Tabelle 3

Verbindungen der Formel

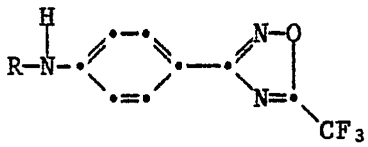

| Verb.Nr. | R | Smp. (°C) |
|---|---|---|
| 3.1 | $CH_3(CH_3O)N-C(O)$ | 88-90 |
| 3.2 | $(CH_3)_2N-C(O)$ | 154-160 |
| 3.3 | $CH_3OC(O)$ | 117-119 |
| 3.4 | $C_2H_5OC(O)$ | 100-103 |
| 3.5 | $(CH_3)_2CHOC(O)$ | 109-112 |
| 3.6 | 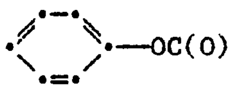 | 116-120 |
| 3.7 | $CH_3-C(O)$ | 170-173 |
| 3.8 | $CH_2=CH-CH_2OC(O)$ | 100-102 |
| 3.9 | $ClCH_2-CH_2OC(O)$ | 115-117 |
| 3.10 | $CH_3NH-C(S)$ | 148-150 |
| 3.11 | $(CH_3)_2N-C(S)$ | 158-159 |
| 3.12 | $CH_3(CH_3O)N-C(S)$ | 142-144 |
| 3.13 | $C_2H_5OC(S)$ | |
| 3.14 | $C_2H_5-C(O)$ | 159-160 |
| 3.15 | $ClCH_2-C(O)$ | 143-144 |
| 3.16 | $C_3H_7NH-C(O)$ | 212-214 |
| 3.17 | $ClCH_2-CH_2NH-C(O)$ | 189-190 |
| 3.18 | $C_2H_5S-C(O)$ | 133-136 |
| 3.19 | $(CH_3)_2CHNH-C(O)$ | 211-212 |
| 3.20 | $C_4H_9NH-C(O)$ | 172-173 |
| 3.21 | $(CH_3)_3CNH-C(O)$ | 160-167 |

Tabelle 4

Verbindungen der Formel

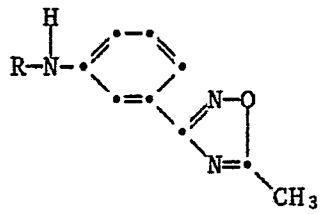

| Verb.Nr. | R | Smp. (°C) |
|---|---|---|
| 4.1 | $CH_3(CH_3O)N-C(O)$ | 74-77 |
| 4.2 | $(CH_3)_2N-C(O)$ | 158-162 |
| 4.3 | $CH_3NH-C(O)$ | 138-140 |
| 4.4 | $C_2H_5-C(O)$ | 113-115 |
| 4.5 | $ClCH_2-C(O)$ | 109-114 |
| 4.6 | $CH_3OC(O)$ | 118-121 |
| 4.7 | $C_2H_5OC(O)$ | 83-85 |
| 4.8 | $(CH_3)_2CHOC(O)$ | 89-92 |
| 4.9 | 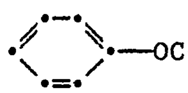OC(O) | 110-112 |
| 4.10 | $CH_2=CH-CH_2OC(O)$ | 79-81 |
| 4.11 | $ClCH_2-CH_2OC(O)$ | 91-93 |
| 4.12 | $CH_3NH-C(S)$ | 128-131 |
| 4.13 | $(CH_3)_2N-C(S)$ | 99-101 |
| 4.14 | $CH_3(CH_3O)N-C(S)$ | 75-77 |
| 4.15 | $C_2H_5OC(S)$ | |
| 4.16 | $(CH_3)_3CNH-C(O)$ | 165-169 |
| 4.17 | $C_4H_9NH-C(O)$ | 106-109 |
| 4.18 | $(CH_3)_2CHNH-C(O)$ | 172-174 |
| 4.19 | $C_3H_7NH-C(O)$ | 132-136 |
| 4.20 | $ClCH_2-CH_2NH-C(O)$ | 121-123 |
| 4.21 | $C_2H_5S-C(O)$ | 114-116 |

2. Formulierungsbeispiele für feste Wirkstoffe der Formel I

(% = Gewichtsprozent)

| 2.1 Spritzpulver | a) | b) | c) |
|---|---|---|---|
| Wirkstoff aus den Tabellen | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.2 Emulsions-Konzentrat | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 2.3 Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit den Träger vermischt und auf einer geeigneten Mühle vermahlen wird.

| 2.4 Extruder Granulat | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses

Gemisch wird extrudiert und anschliessend im Luftstrom getrocknet.

2.5 Umhüllungs-Granulat

| | |
|---|---|
| Wirkstoff aus den Tabellen | 3 % |
| Polyethylenglykol (MG 200) | 3 % |
| Kaolin | 94 % |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.6 Suspensions-Konzentrat

| | |
|---|---|
| Wirkstoff aus den Tabellen | 40 % |
| Ethylenglykol | 10 % |
| Nonylphenolpolyethylenglykolether (15 Mol Ethylenoxid) | 6 % |
| N-Lingninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Formulierungsbeispiele für flüssige Wirkstoffe der Formel I

% = Gewichtsprozent)

| 2.7. Emulsionskonzentrate | a) | b) | c) |
|---|---|---|---|
| Wirkstoff aus den Tabellen | 25 % | 40 % | 50 % |
| Ca-Dodecylbenzolsulfonat | 5 % | 8 % | 6 % |
| Ricinusöl-polyethylenglykolether (36 Mol Ethylenoxid) | 5% | - | - |
| Tributylphenoyl-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.8. Lösungen | a) | b) | c) | d) |
|---|---|---|---|---|
| Wirkstoff aus den Tabellen | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

(MG = Molekulargewicht)

Die Lösungen sind zur Anwendung in Form kleinster Tropfen geeignet.

| 2.9. Granulate | a) | b) |
|---|---|---|
| Wirkstoff aus den Tabellen | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.10. Stäubemittel | a) | b) |
|---|---|---|
| Wirkstoff aus den Tabellen | 2 % | 5 % |
| Hochdisperse Kieselsäure | 1 % | 5 % |
| Talkum | 97 % | - |
| Kaolin | - | 90 % |

Durch inniges Vermischen auf Trägerstoffe mit dem Wirkstoff erhält man gebrauchsfertige Stäubemittel.

## 3. Biologische Beispiele:

### Beispiel 3.1: Wirkung gegen Puccinia graminis auf Weizen

#### a) Residual-protektive Wirkung

Weizenpflanzen werden 6 Tage nach der Aussaat mit einer aus Spritzpulver des Wirkstoffes hergestellten Spritzbrühe (0,006 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

#### b) Systemische Wirkung

Zu Weizenpflanzen wird 5 Tage nach der Aussaat eine aus Spritzpulver des Wirkstoffes hergestellte Spritzbrühe gegossen (0,02 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in

einem Gewächshaus bei ca. 22°C aufgestellt. Die Beurteilung der Rostpustelnentwicklung erfolgt 12 Tage nach der Infektion.

Verbindungen aus den Tabellen zeigen gegen Puccinia-Pilze gute Wirksamkeit. Der Puccinia-Befall wurde durch die Verbindungen Nr. 1.1, 3.1, 3.12 und 4.2 fast vollständig (Befall = 0-10 %) und durch die Verbindungen Nr. 1.14, 1.15, 1.16, 1.18, 1.41, 1.42, 1.43, 2.2, 2.11 und 3.2 zu 80-90 % verhindert. Unbehandelte aber infizierte Kontrollpflanzen weisen dagegen eine Puccinia-Befall von 100 % auf.

Beispiel 3.1: Wirkung gegen Cercospora arachidicola auf Erdnusspflanzen

Residual-protektive Wirkung

10-15 cm hohe Erdnusspflanzen werden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen werden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigen Erdnusspflanzen, die mit Wirkstoffen aus den Tabellen behandelt wurden, einen stark reduzierten Cercospora-Befall. So verhinderten z.B. die Verbindungen Nr. 1.1 und 1.2 das Auftreten von Blattflecken fast vollständig (Befall = 0-10 %).

Beispiel 3.3: Wirkung gegen Erysiphe graminis auf Gerste

a) Residual-protektive Wirkung

Ca. 8 cm hohe Gerstenpflanzen werden mit einer aus Spritzpulver des Wirkstoffes hergestellten Spritzbrühe (0,006 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beuteilt.

Verbindungen aus den Tabellen zeigen gut Wirksamkeit gegen Erysiphe-Pilze. So verhinderte z.B. die Verbindung Nr. 1.23 den Erysiphe-Befall fast vollständig (Befall = 0-10 %). Unbehandelte jedoch infizierte Kontrollpflanzen weisen dagegen einen Befall von 100 % auf.

Beispiel 3.4: Residual-protektive Wirkung gegen Venturia inaequalis auf Apfeltrieben

Apfelstecklinge mit 10-20 cm langen Frischtrieben werden mit einer aus Spritzpulver des Wirkstoffes hergestellten Spritzbrühe (0,02 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24°C aufgestellt. Der Schorfbefall wird 15 Tage nach der Infektion beurteilt. Verbindungen aus den Tabellen zeigen gute Wirksamkeit gegen Venturia. So verhinderte z.B. die Verbindung Nr. 2.12 den Venturia-Befall fast vollständig (Befall = 0-10 %). Unbehandelte jedoch infizierte Triebe weisen dagegen einen 100%igen Befall auf.

## Ansprüche

1. Mittel zur Bekämpfung oder Verhütung eines Befalls von Pflanzen durch Schädlinge, dadurch gekennzeichnet, dass es als aktive Komponente mindestens eine Verbindung der Formel I

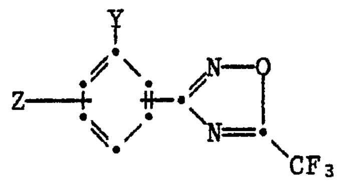

(I)

enthält, worin Y für OR′ oder HNR″ steht, Z Wasserstoff oder $(R_4)_n$bedeutet, und unter welche als Untergruppen die Verbindungen der Formeln I′ und I″

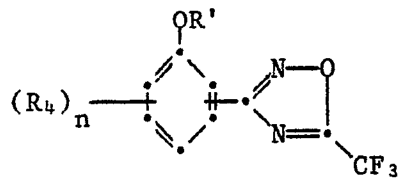

(I′)

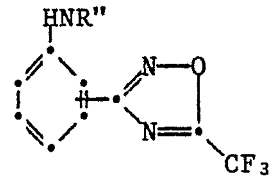

(I″)

fallen, in welchen bedeuten:

R′ Wasserstoff, $C_1$-$C_4$-Alkyl, Aryl, substituiertes Aryl, $C(X)NR_1'(R_2')$, $C(O)XR_3'$ oder $COR_3'$;
$R_1'$ $C_1$-$C_9$-Alkyl, $C_1$-$C_4$-Alkoxy, $C_2$-$C_4$-Alkenyl, Aryl oder substituiertes Aryl;
$R_2'$ Wasserstoff oder $C_1$-$C_4$-Alkyl;
$R_3'$ $C_1$-$C_{15}$-Alkyl, $C_2$-$C_4$-Alkoxy oder Aryl;
$R_4$ Halogen, $C_1$-$C_4$-Alkoxy oder Nitro; und
n 0, 1 oder 2;
X Sauerstoff oder Schwefel;
R″ $C(O)NR''(R_2'')$, $C(S)NR_1''(R_2'')$, $COOR_3''$, $COSR_3''$ oder $COR_3''$;
$R_1''$ $C_1$-$C_4$-Alkyl, $C_1$-$C_4$-Alkoxy oder Aryl;
$R_2''$ Wasserstoff oder $C_1$-$C_4$-Alkyl und
$R_3''$ $C_1$-$C_9$-Alkyl, $C_1$-$C_4$-Haloalkyl, $C_3$-$C_4$-Alkenyl oder Aryl;
einschliesslich ihrer Säureadditionssalze.

2. Mittel gemäss Anspruch 1, dadurch gekennzeichnet, dass es als aktive Komponente mindestens eine Verbindung der Formel I′ enthält,

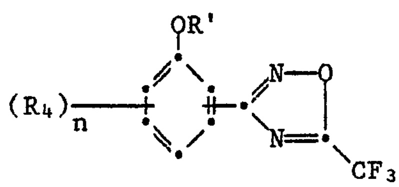

(I′),

in welcher

R′ für Wasserstoff, $C_1$-$C_4$-Alkyl, Aryl, substituiertes Aryl, $C(X)NR_1'(R_2')$, $C(O)XR_3'$ oder $COR_3'$ steht;
$R_1'$ $C_1$-$C_9$-Alkyl, $C_1$-$C_4$-Alkoxy, $C_2$-$C_4$-Alkenyl, Aryl oder substituiertes Aryl;
$R_2'$ Wasserstoff oder $C_1$-$C_4$-Alkyl;
$R_3'$ $C_1$-$C_{15}$-Alkyl, $C_2$-$C_4$-Alkenyl oder Aryl
$R_4$ Halogen, $C_1$-$C_4$-Alkoxy oder Nitro bedeuten; und
n 0, 1 oder 2;
X Sauerstoff oder Schwefel darstellt, einschliesslich ihrer Säureadditionssalze

3. Verbindungen der Formel I′

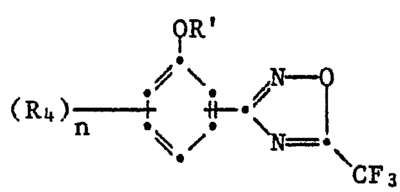

(I′),

in welcher
R' für Wasserstoff, $C_1$-$C_4$-Alkyl, Aryl, substituiertes Aryl, C(X)NR$_1$'(R$_2$'), C(O)XR$_3$' oder COR$_3$' steht;
R$_1$' $C_1$-$C_9$-Alkyl, $C_1$-$C_4$-Alkoxy, $C_2$-$C_4$-Alkenyl, Aryl oder substituiertes Aryl;
R$_2$' Wasserstoff oder $C_1$-$C_4$-Alkyl;
R$_3$' $C_1$-$C_{15}$-Alkyl, $C_2$-$C_4$-Alkenyl oder Aryl
R$_4$ Halogen, $C_1$-$C_4$-Alkoxy oder Nitro bedeuten; und
n 0, 1 oder 2;
X Sauerstoff oder Schwefel darstellt, einschliesslich ihrer Säureadditionsalze

4. Verbindungen gemäss Anspruch 3 der Formel Ia'

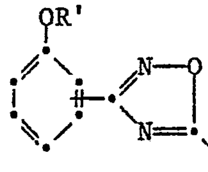

(Ia'),

in welcher
R' für Wasserstoff, $C_1$-$C_4$-Alkyl, Aryl, substituiertes Aryl, C(X)NR$_1$'(R$_2$'), C(O)XR$_3$' oder COR$_3$' steht;
R$_1$' $C_1$-$C_9$-Alkyl, $C_1$-$C_4$-Alkoxy, $C_2$-$C_4$-Alkenyl, Aryl oder substituiertes Aryl;
R$_2$' Wasserstoff oder $C_1$-$C_4$-Alkyl;
R$_3$' $C_1$-$C_{15}$-Alkyl, $C_2$-$C_4$-Alkenyl oder Aryl
und
X Sauerstoff oder Schwefel darstellt, einschliesslich ihrer Säureadditionsalze.

5. Verbindungen der Formel Ia' gemäss Anspruch 4, in welcher R' für Wasserstoff, Phenyl, substituiertes Phenyl, C(X)NR$_1$'(R$_2$'), C(O)XR$_3$' oder COR$_3$' steht;
R$_1$' $C_1$-$C_9$-Alkyl, $C_1$-$C_4$-Alkoxy, $C_2$-$C_4$-Alkenyl, Aryl oder Phenyl;
R$_2$' Wasserstoff oder $C_1$-$C_4$-Alkyl;
R$_3$' $C_1$-$C_{15}$-Alkyl, $C_2$-$C_4$-Alkenyl oder Phenyl bedeuten; und
X Sauerstoff oder Schwefel darstellt.

6. Verbindungen der Formel Ia' Anspruch 4, in welcher der Rest

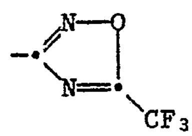

in 4-Stellung des Phenylrings steht,
R' für Wasserstoff, Phenyl, durch Halogen oder Methyl substituiertes Phenyl, C(X)NR$_1$'(R$_2$'), C(O)XR$_3$' oder COR$_3$' steht, wobei R$_1$' $C_1$-$C_4$-Alkyl, $C_1$-$C_2$-Alkoxy, Allyl oder Phenyl; R$_2$' Wasserstoff oder $C_1$-$C_2$-Alkyl; R$_3$' $C_1$-$C_9$-Alkyl, $C_2$-$C_4$-Alkenyl oder Phenyl bedeuten; und
X Sauerstoff oder Schwefel darstellt.

7. Verbindungen der Formel Ia' gemäss Anspruch 4, in welcher der Rest

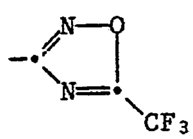

in 4-Stellung des Phenylrings steht, R' Wasserstoff, $C_1$-$C_4$-Alkyl, Phenyl, substituiertes Phenyl, C(X)NR$_1$'(R$_2$'), C(O)XR$_3$' oder COR$_3$'; R$_1$' $C_1$-$C_4$-Alkyl, $C_1$-$C_4$-Alkoxy, Allyl oder Phenyl; R$_2$' Wasserstoff oder $C_1$-$C_4$-Alkyl; R$_3$' $C_1$-$C_9$-Alkyl, $C_2$-$C_4$-Alkenyl oder Phenyl bedeuten; und X Sauerstoff oder Schwefel darstellt.

8. Eine Verbindung der Formel Ia' gemäss Anspruch 4 aus der Gruppe: 3-(4-Methylaminocarbonyloxyphenyl)-5-trifluormethyl-1,2,4-oxadiazol;
3-(4-Isopropylaminocarbonyloxyphenyl)-5-trifluormethyl-1,2,4-oxadiazol;
3-(4-Ethylaminocarbonyloxyphenyl)-5-trifluormethyl-1,2,4-oxadiazol;
3-(4-Allylaminocarbonyloxyphenyl)-5-trifluormethyl-1,2,4-oxadiazol;

3-[4-(2-Chlor-4-trifluormethylphenoxy)-phenyl]-5-trifluormethyl-1,2,4-oxadiazol;
3-[Methoxy-(methyl)-aminocarbonyloxyphenyl]-5-trifluormethyl-1,2,4-oxadiazol;
3-(4-Hexanoyloxyphenyl)-5-trifluormethyl-1,2,4-oxadiazol;
3-[4-(Phenylaminocarboxy)-phenyl]-5-trifluormethyl-1,2,4-oxadiazol;
3-[4-(2-Methyl-3-chlorphenylaminocarboxy)-phenyl]-5-trifluormethyl-1,2,4-oxadiazol;
3-[4-(3-Ethylphenylaminocarboxyphenyl)]-5-trifluormethyl-1,2,4-oxadiazol.

9. Mittel gemäss Anspurch 1, dadurch gekennzeichnet, dass es als aktive Komponente mindestens eine Verbindung der Formel I" enthält,

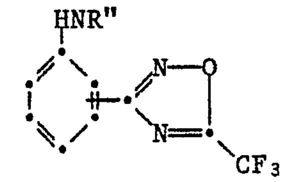

(I"),

in welcher
R" $C(O)NR_1''(R_2'')$, $C(S)NR_1''(R_2'')$, $COOR_3''$, $COSR_3''$ oder $COR_3''$;
$R_1''$ $C_1$-$C_4$-Alkyl, $C_1$-$C_4$-Alkoxy oder Aryl;
$R_2''$ Wasserstoff oder $C_1$-$C_4$-Alkyl und
$R_3''$ $C_1$-$C_9$-Alkyl, $C_1$-$C_4$-Haloalkyl, $C_3$-$C_4$-Alkenyl oder Aryl bedeuten
einschliesslich ihrer Säureadditionssalze.

10. Verbindungen der Formel Ia"

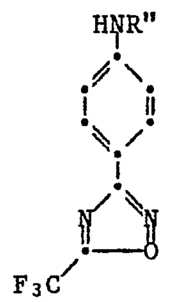

(Ia"),

in welcher
R" $C(O)NR_1''(R_2'')$, $C(S)NR_1''(R_2'')$, $COOR_3''$, $COSR_3''$ oder $COR_3''$;
$R_1''$ $C_1$-$C_2$-Alkyl, $C_1$-$C_2$-Alkoxy oder Phenyl;
$R_2''$ Wasserstoff oder $C_1$-$C_2$-Alkyl und
$R_3''$ $C_1$-$C_3$-Alkyl, $C_1$-$C_2$-Haloalkyl, $C_3$-$C_4$-Alkenyl oder Phenyl bedeuten.

12. Eine Verbindungen der Formel Ia" gemäss Anspruch 10 aus der Gruppe:
3-[4-(3-Methyl-3-methoxyureido)-phenyl]-5-trifluormethyl-1,2,4-oxadiazol;
3-[4-(3,3-Dimethylureido)-phenyl]-5-trifluormethyl-1,2,4-oxadiazol;
3-(4-Methyoxycarbonylaminophenyl)-5-trifluormethyl-1,2,4-oxadiazol;
3-(4-Ethoxycarbonylaminophenyl)-5-trifluormethyl-1,2,4-oxadiazol;
3-(4-Isopropoxycarbonylaminophenyl)-5-trifluormethyl-1,2,4-oxadiazol;
3-(4-Phenyloxycarbonylaminophenyl)-5-trifluormethyl-1,2,4-oxadiazol;
3-(4-Acetylaminophenyl)-5-trifluormethyl-1,2,4-oxadiazol;
3-(4-Allyloxycarbonylaminophenyl)-5-trifluormethyl-1,2,4-oxadiazol;
3-[4-(2-Chlorethyloxycarbonylamino)-phenyl]-5-trifluormethyl-1,2,4-oxadiazol;
3-[4-(3-Methylthioureido)-phenyl]-5-trifluormethyl-1,2,4-oxadiazol;
3-[4-(3,3-Dimethylthioureido)-phenyl]-5-trifluormethyl-1,2,4-oxadiazol;
3-[4-(3-Methyl-3-methoxythioureido)-phenyl]-5-trifluormethyl-1,2,4-oxadiazol;
3-(4-Ethylthiocarbonylaminophenyl)-5-trifluormethyl-1,2,4-oxadiazol;
3-(4-Ethylcarbonylaminophenyl)-5-trifluormethyl-1,2,4-oxadiazol;
3-(4-Chlormethylcarbonylaminophenyl)-5-trifluormethyl-1,2,4-oxadiazol.

13. Mittel nach Anspruch 1, dadurch gekennzeichnet, dass es als aktive Komponente mindestens eine Verbindung enthält, ausgewählt aus der Gruppe:
3-[3-(3,3-Dimethylureido)-phenyl]-5-trifluormethyl-1,2,4-oxadiazol;

3-[3-(3-Methyl-3-methoxyureido)-phenyl]-5-trifluormethyl-1,2,4-oxadiazol;
3-[3-(3-Methylureido)-phenyl]-5-trifluormethyl-1,2,4-oxadiazol;
3-(3-Ethoxycarbonylaminophenyl)-5-trifluormethyl-1,2,4-oxadiazol;
3-(3-Chlormethoxycarbonylaminophenyl)-5-trifluormethyl-1,2,4-oxadiazol;
3-(3-Methoxycarbonylaminophenyl)-5-trifluormethyl-1,2,4-oxadiazol;
3-(3-Isopropoxycarbonylaminophenyl)-5-trifluormethyl-1,2,4-oxadiazol;
3-(3-Phenoxycarbonylaminophenyl)-5-trifluormethyl-1,2,4-oxadiazol;
3-(3-Ally(oxycarbonylaminophenyl)-5-trifluormethyl-1,2,4-oxadiazol;
oder
3-[3-(2-Chlorethoxycarbonylamino)-phenyl]-5-trifluormethyl-1,2,4-oxadiazol.

14. Verfahren zur Herstellung der Verbindungen der Formel I gemäss Anspruch 1, dadurch gekennzeichnet, dass

1. für die Verbindungen der Formel I' ein Oximderivat der Formel II'

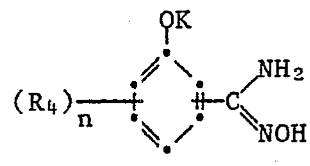

(II')

mit Trifluoressigsäureanhydrid zu einem Phenolderivat der Formel Ib'

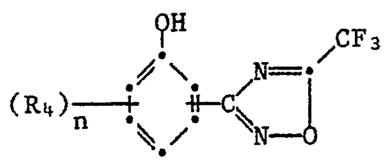

(Ib')

umgesetzt wird, welches dann zur weiteren Substitution

1.1 mit einem Carbamoylchlorid der Formel III'

$R_1'(R_2')N\text{-}CXCl$ (III')

oder

1.2 mit einem Chlorameisensäureester der Formel IIIa'

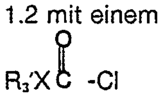

(IIIa')

oder

1.3 mit einem Carbonsäurechlorid der Formel IIIb'

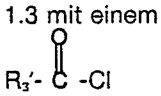

(IIIb')

oder

1.4 mit Dialkylsulfat oder Alkylhalogenid, vorzugsweise Alkylbromid oder Alkyljodid, mit 1 bis 4 Kohlenstoffatomen in den Alkylgruppen in Gegenwart einer Base als Säureakzeptor zur Reaktion gebracht wird, wobei X Sauerstoff oder Schwefel bedeutet und $R_1'$, $R_2'$, $R_3'$, $R_4$ und n die unter Formel I angegebenen Bedeutungen besitzen,

oder

1.5 ein Oximderivat der Formel IIa'

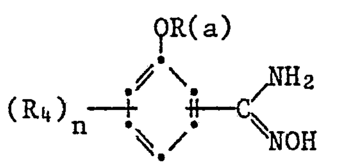

(IIa')

mit Trifluoressigsäureanhydrid zu einem Phenolderivat der Formel Ic'

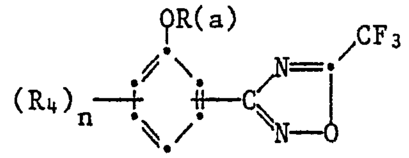

(Ic') ,

worin R(a) Aryl oder substituiertes Aryl darstellte und $R_4$ und n die unter Formel I angegebenen Bedeutungen besitzen, zur Reaktion gebracht wird und

2. für die Verbindungen der Formel I" Ein Anilinderivat der Formel II"

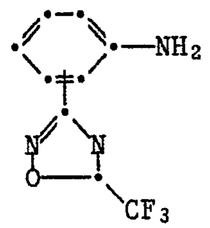

(II")

2.1 mit einem Carbamoylchlorid der Formel III"

$R_1''(R_2'')N\text{-}CXCl$ (III")

oder

2.2 mit einem Chlorameisensäureester der Formel IIIa"

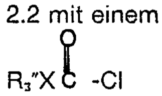 (IIIa")

oder

2.3 mit einem Carbonsäurechlorid der Formel IIIb"

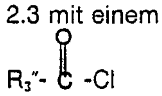 (IIIb")

in einem inerten Lösungsmittel in Gegenwart einer Base als Säureakzeptor

oder

2.4 mit einem Isocyanatderivat der Formel IV

$R_1''\text{-}N=C=X$ (IV)

in einem inerten Lösungsmittel umgesetzt, wobei X Sauerstoff oder Schwefel bedeutet und $R_1''$, $R_2''$ und $R_3''$ die unter Formel I" angegebenen Bedeutungen besitzen.

15. Mittel nach Anspruch 1, dadurch gekennzeichnet, dass es 0,1 bis 99 % eines Wirkstoffs der Formel I, 99,9 bis 1 % eines festen oder flüssigen Zusatzstoffes und 0 bis 25 % eines Tensides enthält.

16. Mittel nach Anspruch 15, dadurch gekennzeichnet, dass es 0,1 bis 95 % eines Wirkstoffs der Formel I, 99,9 bis 5 % eines festen oder flüssigen Zusatzstoffes und 0,1 bis 25 % eines Tensides enthält.

17. Verfahren zur Herstellung eines agrochemischen Mittels, dadurch gekennzeichnet, dass man mindestens eine Verbindung der Foreml I aus einem der Ansprüche 1 bis 13 mit geeigneten festen oder flüssigen Zusatzstoffen und Tensiden innig vermischt.

18. Verfahren zur Bekämpfung oder Verhütung eines Befalls von Kulturpflanzen durch phytopathogene Mikroorganismen, dadurch gekennzeichnet, dass man eine Verbindung der Formel I aus einem der Ansprüche 1 bis 13 auf die Pflanze oder deren Standort appliziert.

19. Verwendung von Verbindungen der Formel 1 aus einem der Ansprüche 1 bis 13 zur Bekämpfung und/oder zur präventiven Verhütung eines Befalls von Mikroorganismen.

20. Verwendung gemäss Anspruch 19, dadurch gekennzeichnet, dass es sich bei den Mikroorganismen um phytopathogene Pilze handelt.

Patentansprüche für den folgenden Vertragsstaat: ES

1. Verfahren zur Herstellung der Verbindungen der Formel I

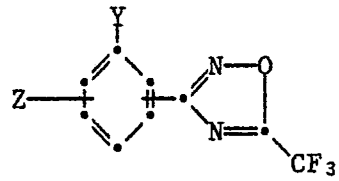

(I),

worin Y für OR′ oder HNR″ steht, Z Wasserstoff oder $(R_4)_n$ bedeutet, und unter welche als Untergruppen die Verbindungen der Formeln I′ und I″

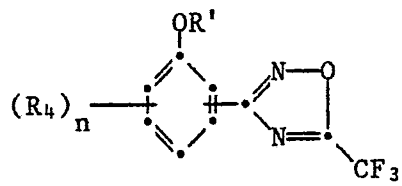

(I′)

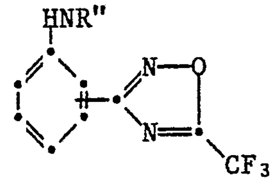

(I″)

fallen, in welchen bedeuten:
R′ Wasserstoff, $C_1$-$C_4$-Alkyl, Aryl, substituiertes Aryl, $C(X)NR_1'(R_2')$, $C(O)XR_3'$ oder $COR_3'$;
$R_1'$ $C_1$-$C_9$-Alkyl, $C_1$-$C_4$-Alkoxy, $C_2$-$C_4$-Alkenyl, Aryl oder substituiertes Aryl;
$R_2'$ Wasserstoff oder $C_1$-$C_4$-Alkyl;
$R_3'$ $C_1$-$C_{15}$-Alkyl, $C_2$-$C_4$-Alkoxy oder Aryl;
$R_4$ Halogen, $C_1$-$C_4$-Alkoxy oder Nitro; und
n 0, 1 oder 2;
X Sauerstoff oder Schwefel;
R″ $C(O)NR''(R_2'')$, $C(S)NR_1''(R_2'')$, $COOR_3''$, $COSR_3''$ oder $COR_3''$;
$R_1''$ $C_1$-$C_4$-Alkyl, $C_1$-$C_4$-Alkoxy oder Aryl;
$R_2''$ Wasserstoff oder $C_1$-$C_4$-Alkyl und
$R_3''$ $C_1$-$C_9$-Alkyl, $C_1$-$C_4$-Haloalkyl, $C_3$-$C_4$-Alkenyl oder Aryl; einschliesslich ihrer Säureadditionssalze, dadurch gekennzeichnet, dass

1. für die Verbindungen der Formel I′
ein Oximderivat der Formel II′

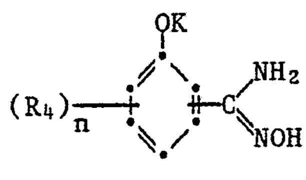

(II′)

mit Trifluoressigsäureanhydrid zu einem Phenolderivat der Formel Ib′

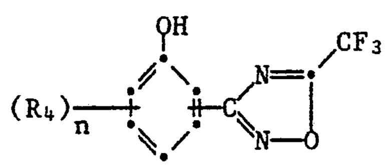

(Ib′)

umgesetzt wird, welches dann zur weiteren Substitution
1.1 mit einem Carbamoylchlorid der Formel III′
$R_1'(R_2')N$-CXCl (III′)
oder
1.2 mit einem Chlorameisensäureester der Formel IIIa′

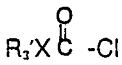 (IIIa')

oder

1.3 mit einem Carbonsäurechlorid der Formel IIIb'

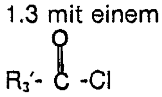 (IIIb')

oder

1.4 mit Dialkylsulfat oder Alkyhalogenid, vorzugsweise Alkylbromid oder Alkyljodid, mit 1 bis 4 Kohlenstoffatomen in den Alkylgruppen in Gegenwart einer Base als Säureakzeptor zur Reaktion gebracht wird, wobei X Sauerstoff oder Schwefel bedeutet und $R_1'$, $R_2'$, $R_3'$, $R_4$ und n die unter Formel I angegebenen Bedeutungen besitzen, oder

1.5 ein Oximderivat der Formel IIa'

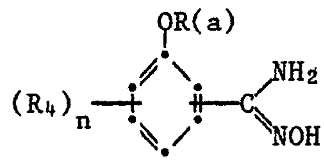 (IIa')

mit Trifluoressigsäureanhydrid zu einem Phenolderivat der Formel Ic'

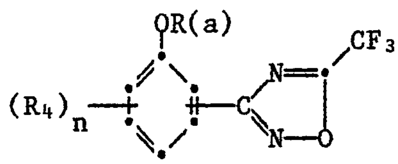 (Ic') ,

worin R(a) Aryl oder substituiertes Aryl darstellte und $R_4$ und n die unter Formel I angegebenen Bedeutungen besitzen, zur Reaktion gebracht wird und

2. für die Verbindungen der Formel I" ein Anilinderivat der Formel II"

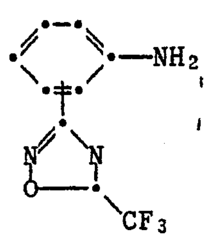 (II")

2.1 mit einem Carbamoylchlorid der Formel III"

$R_1''(R_2'')N\text{-}CXCl$ (III")

oder

2.2 mit einem Chlorameisensäureester der Formel IIIa"

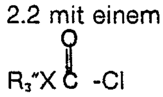 (IIIa")

oder

2.3 mit einem Carbonsäurechlorid der Formel IIIb"

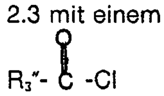 (IIIb")

in einem inerten Lösungsmittel in Gegenwart einer Base als Säureakzeptor

oder

2.4 mit einem Isocyanatderivat der Formel IV

$R_1''\text{-}N=C=X$ (IV)

in einem inerten Lösungsmittel umgesetzt, wobei X Sauerstoff oder Schwefel bedeutet und $R_1''$, $R_2''$ und $R_3''$ die unter Formel I" angegebenen Bedeutungen besitzen.

2. Verfahren gemäss Anspruch 1 dadurch gekennzeichnet, dass Verbindungen der Formel I'

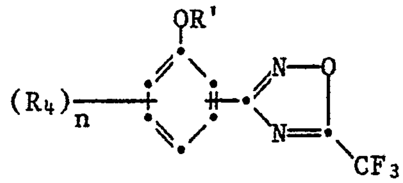

(I')

hergestellt werden, in welcher
R' für Wasserstoff, $C_1$-$C_4$-Alkyl, Aryl, substituiertes Aryl, $C(X)NR_1'(R_2')$, $C(O)XR_3'$ oder $COR_3'$ steht;
$R_1'$ $C_1$-$C_9$-Alkyl, $C_1$-$C_4$-Alkoxy, $C_2$-$C_4$-Alkenyl, Aryl oder substituiertes Aryl;
$R_2'$ Wasserstoff oder $C_1$-$C_4$-Alkyl;
$R_3'$ $C_1$-$C_{15}$-Alkyl, $C_2$-$C_4$-Alkenyl oder Aryl
$R_4$ Halogen, $C_1$-$C_4$-Alkoxy oder Nitro bedeuten; und
n 0, 1 oder 2;
X Sauerstoff oder Schwefel darstellt, einschliesslich ihrer Säureadditionsalze

3. Verfahren gemäss Anspruch 1 dadurch gekennzeichnte, dass Verbindungen der Formel Ia'

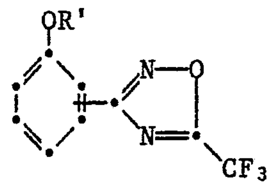

(Ia')

hergestellt werden, in welcher
R' für Wasserstoff, $C_1$-$C_4$-Alkyl, Aryl, substituiertes Aryl, $C(X)NR_1'(R_2')$, $C(O)XR_3'$ oder $COR_3'$ steht;
$R_1'$ $C_1$-$C_9$-Alkyl, $C_1$-$C_4$-Alkoxy, $C_2$-$C_4$-Alkenyl, Aryl oder substituiertes Aryl;
$R_2'$ Wasserstoff oder $C_1$-$C_4$-Alkyl;
$R_3'$ $C_1$-$C_{15}$-Alkyl, $C_2$-$C_4$-Alkenyl oder Aryl
und
X Sauerstoff oder Schwefel darstellt, einschliesslich ihrer Säureadditionsalze.

4. Verfahren gemäss Anspruch 1 dadurch gekennzeichnet, dass Verbindungen der Formel Ia' hergestellt werden, in welcher
R' für Wasserstoff, Phenyl, substituiertes Phenyl, $C(X)NR_1'(R_2')$, $C(O)XR_3'$ oder $COR_3'$ steht;
$R_1'$ $C_1$-$C_9$-Alkyl, $C_1$-$C_4$-Alkoxy, $C_2$-$C_4$-Alkenyl, Aryl oder Phenyl;
$R_2'$ Wasserstoff oder $C_1$-$C_4$-Alkyl;
$R_3'$ $C_1$-$C_{15}$-Alkyl, $C_2$-$C_4$-Alkenyl oder Phenyl bedeuten; und
X Sauerstoff oder Schwefel darstellt.

5. Verfahren gemäss Anspruch 1 dadurch gekennzeichnet, dass Verbindungen der Formel Ia' hergestellt werden, in welcher der Rest

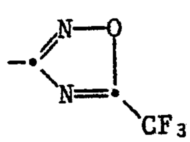

in 4-Stellung des Phenylrings steht,
R' für Wasserstoff, Phenyl, durch Halogen oder Methyl substituiertes Phenyl, $C(X)NR_1'(R_2')$, $C(O)XR_3'$ oder $COR_3'$ steht, wobei $R_1'$ $C_1$-$C_4$-Alkyl, $C_1$-$C_2$-Alkoxy, Allyl oder Phenyl; $R_2'$ Wasserstoff oder $C_1$-$C_2$-Alkyl; $R_3'$ $C_1$-$C_9$-Alkyl, $C_2$-$C_4$-Alkenyl oder Phenyl bedeuten; und
X Sauerstoff oder Schwefel darstellt.

6. Verfahren gemäss Anspruch dadurch gekennzeichnet, dass Verbindungen der Formel Ia' hergestellt werden, in welcher der Rest

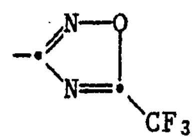

in 4-Stellung des Phenylrings steht, R′ Wasserstoff, $C_1$-$C_4$-Alkyl, Phenyl, substituiertes Phenyl, C(X)NR$_1$′)R$_2$′), C(O)XR$_3$′ oder COR$_3$′; R$_1$′ $C_1$-$C_4$-Alkyl, $C_1$-$C_4$-Alkoxy, Allyl oder Phenyl; R$_2$′ Wasserstoff oder $C_1$-$C_4$-Alkyl; R$_3$′ $C_1$-$C_9$-Alkyl, $C_2$-$C_4$-Alkenyl oder Phenyl bedeuten; und X Sauerstoff oder Schwefel darstellt.

7. Verfahren gemäss Anspruch 1 dadurch gekennzeichnet, dass eine Verbindung der Formel Ia′ aus der Gruppe hergestellt wird:
3-(4-Methylaminocarbonyloxyphenyl)-5-trifluormethyl-1,2,4-oxadiazol;
3-(4-Isopropylaminocarbonyloxyphenyl)-5-trifluormethyl-1,2,4-oxadiazol;
3-(4-Ethylaminocarbonyloxyphenyl)-5-trifluormethyl-1,2,4-oxadiazol;
3-(4-Allylaminocarbonyloxyphenyl)-5-trifluormethyl-1,2,4-oxadiazol;
3-[4-(2-Chlor-4-trifluormethylphenoxy)-phenyl]-5-trifluormethyl-1,2,4-oxadiazol;
3-[Methoxy-(methyl)-aminocarbonyloxyphenyl]-5-trifluormethyl-1,2,4-oxadiazol;
3-(4-Hexanoyloxyphenyl)-5-trifluormethyl-1,2,4-oxadiazol;
3-[4-(Phenylaminocarboxy)-phenyl]-5-trifluormethyl-1,2,4-oxadiazol;
3-[4-(2-Methyl-3-chlorphenylaminocarboxy)-phenyl]-5-trifluormethyl-1,2,4-oxadiazol;
3-[4-(3-Ethylphenylaminocarboxyphenyl)]-5-trifluormethyl-1,2,4-oxadiazol.

8. Verfahren gemäss Anspurch 1, dadurch gekennzeichnet, dass Verbindungen der Formel Ia″

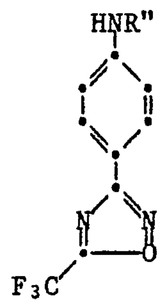

(Ia″)

hergestellt werden, in welcher
R″ C(O)NR$_1$″(R$_2$″), C(S)NR$_1$″(R$_2$″), COOR$_3$″, COSR$_3$″ oder COR$_3$″;
R$_1$″ $C_1$-$C_4$-Alkyl, $C_1$-$C_4$-Alkoxy oder Aryl;
R$_2$″ Wasserstoff oder $C_1$-$C_4$-Alkyl und
R$_3$″ $C_1$-$C_9$-Alkyl, $C_1$-$C_4$-Haloalkyl, $C_3$-$C_4$-Alkenyl oder Aryl bedeuten.

9. Verfahren gemäss Anspruch 1 dadurch gekennzeichnet, dass Verbindungen der Formel Ia″ hergestellt werden, in welcher
R″ C(O)NR$_1$″(R$_2$″), C(S)NR$_1$″(R$_2$″), COOR$_3$″, COSR$_3$″ oder COR$_3$″;
R$_1$″ $C_1$-$C_2$-Alkyl, $C_1$-$C_2$-Alkoxy oder Phenyl;
R$_2$″ Wasserstoff oder $C_1$-$C_2$-Alkyl und
R$_3$″ $C_1$-$C_3$-Alkyl, $C_1$-$C_2$-Haloalkyl, $C_3$-$C_4$-Alkenyl oder Phenyl bedeuten.

10. Verfahren gemäss Anspruch 1 dadurch gekennzeichnet, dass eine Verbindungen der Formel Ia″ aus der Gruppe hergestellt wird:
3-[4-(3-Methyl-3-methoxyureido)-phenyl]-5-trifluormethyl-1,2,4-oxadiazol;
3-[4-(3,3-Dimethylureido)-phenyl]-5-trifluormethyl-1,2,4-oxadiazol;
3-(4-Methyoxycarbonylaminophenyl)-5-trifluormethyl-1,2,4-oxadiazol;
3-(4-Ethoxycarbonylaminophenyl)-5-trifluormethyl-1,2,4-oxadiazol;
3-(4-Isopropoxycarbonylaminophenyl)-5-trifluormethyl-1,2,4-oxadiazol;
3-(4-Phenyloxycarbonylaminophenyl)-5-trifluormethyl-1,2,4-oxadiazol;
3-(4-Acetylaminophenyl)-5-trifluormethyl-1,2,4-oxadiazol;
3-(4-Allyloxycarbonylaminophenyl)-5-trifluormethyl-1,2,4-oxadiazol;
3-[4-(2-Chlorethyloxycarbonylamino)-phenyl]-5-trifluormethyl-1,2,4-oxadiazol;
3-[4-(3-Methylthioureido)-phenyl]-5-trifluormethyl-1,2,4-oxadiazol;
3-[4-(3,3-Dimethylthioureido)-phenyl]-5-trifluormethyl-1,2,4-oxadiazol;
3-[4-(3-Methyl-3-methoxythioureido)-phenyl]-5-trifluormethyl-1,2,4-oxadiazol;

3-(4-Ethylthiocarbonylaminophenyl)-5-trifluormethyl-1,2,4-oxadiazol;
3-(4-Ethylcarbonylaminophenyl)-5-trifluormethyl-1,2,4-oxadiazol;
3-(4-Chlormethylcarbonylaminophenyl)-5-trifluormethyl-1,2,4-oxadiazol.